# EUROPÄISCHE PATENTSCHRIFT

(11) **EP 3 566 725 B1**
(45) Veröffentlichungstag und Bekanntmachung des Hinweises auf die Patenterteilung: **27.10.2021**
(21) Anmeldenummer: 18171026.0
(22) Anmeldetag: 07.05.2018
(51) Int. Cl.: A61L 27/04, A61L 27/06, A61L 27/10, A61L 27/30, A61L 27/54

(54) **ANTIMIKROBIELLE IMPLANTATBESCHICHTUNG**
ANTIMICROBIAL IMPLANT COATING
REVÊTEMENT D'IMPLANT ANTIMICROBIEN

(43) Veröffentlichungstag der Anmeldung: 13.11.2019
(73) Patentinhaber: Waldemar Link GmbH & Co. KG, 22339 Hamburg (DE)
(72) Erfinder: LINK, Helmut D., 22397 Hamburg (DE); CSASZAR, Richard, 23795 Bad Segeberg (DE)
(74) Vertreter: Hoffmann Eitle

(56) Entgegenhaltungen:
- EP-A1- 2 444 108
- WO-A2-2009/094684
- DE-A1- 2 647 088
- US-A1- 2009 198 343
- CLAUS MOSEKE ET AL: "Hard implant coatings with antimicrobial properties", JOURNAL OF MATERIALS SCIENCE: MATERIALS IN MEDICINE, KLUWER ACADEMIC PUBLISHERS, BO, Bd. 22, Nr. 12, 16. Oktober 2011 (2011-10-16), Seiten 2711-2720, XP019985467, ISSN: 1573-4838, DOI: 10.1007/S10856-011-4457-6
- DATABASE WPI Week 201717 Thomson Scientific, London, GB; AN 2017-00457E XP002786273, & CN 106 175 996 A (TIANJIN UROLOGICAL SURGICAL DEPT INST) 7. Dezember 2016 (2016-12-07) -& CN 106 175 996 A (TIANJIN MEDICAL UNIV; TIANJIN INST OF UROLOGY; TIANJIN WENTUO TECH DEV) 7. Dezember 2016 (2016-12-07)
- T DE LOS ARCOS ET AL: "Preparation and characterization of TiN-Ag nanocomposite films", VACUUM., Bd. 67, Nr. 3-4, 1. September 2002 (2002-09-01), Seiten 463-470, XP055353364, GB ISSN: 0042-207X, DOI: 10.1016/S0042-207X(02)00232-4

## Beschreibung

### GEBIET DER ERFINDUNG

Die Erfindung betrifft eine Beschichtung für Wirbelsäulenimplantatkomponenten und ein Verfahren zum Auftragen dieser Beschichtung.

### STAND DER TECHNIK

Einer der Gründe für das Versagen eines Implantats in situ ist eine Infektion mit Erregern. Erreger, die solch ein Versagen hervorrufen können, sind vor allem Staphylokokken, wie zum Beispiel Staphylococcus epidermidis. Diese besiedeln die Haut und Schleimhäute eines Menschen. Des Weiteren hat zum Beispiel unter anderem der Erreger Staphylococcus epidermidis die Fähigkeit, sich mit Hilfe eines Biofilms auf einer Implantatoberfläche anzusiedeln. In diesem Biofilm ist dieser Erreger vor Antibiotika, Phagozyten sowie anderen Immunantworten des Körpers geschützt. Es gibt Hinweise darauf, dass Staphylococcus epidermidis nach der Implantation von Implantaten eine häufige Ursache für postoperative Infektionen ist.

Wird so eine Infektion festgestellt, wird im Allgemeinen versucht, diese durch Wirkstoffe, wie zum Beispiel Antibiotika, zurückzudrängen. Zeigt dieses Vorgehen keinen Erfolg, kann es allerdings notwendig werden, das Implantat wieder zu explantieren. Durch die Entnahme wird zumindest ein Teil des Infektionsherds entnommen. Zwar führt dieses Vorgehen zu einer leichteren Behandlung der Infektion, hat aber eine verminderte Mobilität des Patienten zur Folge.

Im Fall einer Infektion mit Staphylococcus epidermidis kommt erschwerend hinzu, dass dieser Keim regelmäßig Antibiotikaresistenzen aufweist (80% nach Takizawa et al. in SPINE Volume 42, Nr. 7, pp 525-530). Nach Takizawa gibt es im Bereich der Wirbelsäulenchirurgie Anzeichen, dass die als Methicillin-resistente Staphylococcus epidermidis (MRSE) bekannten Erreger wegen ihrer geringeren Virulenz weniger Infektionszeichen zeigen und damit das Risiko in sich bergen, dass eine Infektion erst spät erkannt wird. Dies kann für einen Patienten im schlimmsten Fall postoperativ zu einer höheren Morbidität führen als präoperativ, insbesondere wenn durch die Infektion die Verwendung eines Implantats nicht mehr möglich ist.

Um solche Komplikationen zu umgehen, ist es deswegen erstrebenswert, bereits der Ansiedlung von Erregern entgegenzuwirken. So schlägt die US 2009/0198343 A1 vor, eine Beschichtung für ein künstliches Gelenk, die für die Laufflächen von Metallpaarungen vorgesehen ist, mit einer antimikrobiellen Wirkung zu versehen. Um dies zu erreichen, wird in der US 2009/0198343 A1 eine Chromnitridbeschichtung durch Silber ergänzt, indem die Reibfläche des Implantats abwechselnd mit Chromnitrid und Silber beschichtet wird. Jedoch ist Chromnitrid als sensibilisierend eingestuft, sodass nach Implantation die Gefahr besteht, dass allergische Reaktionen hervorgerufen werden. Zudem ist die in der US 2009/0198343 A1 offenbarte Beschichtung nur sehr eingeschränkt gegen eine Ansiedlung von Staphylococcus epidermidis wirksam.

Weiterhin offenbart die EP 2 444 108 A1 eine Prothesenkomponente mit einer Gleitfläche, die eine Titannitridbeschichtung mit eingeschlossenen Silberatomen aufweist, wobei der Anteil an Silber 2-15 Gew.-% beträgt.

Moseke et al. beschäftigen sich in ihrer Studie mit einer TiN/Ag-Beschichtung für orthopädische Implantate (Claus Moseke et al.: "Hard implant coatings with antimicrobial properties" in Journal of Materials Science: Materials in Medicine, Kluwer Academic Publishers, BO, Bd. 22, Nr. 12, pp. 2711-20). Der Silberanteil betrug 1,4 % oder 4,6 % und zeigte bei Staphylococcus epidermidis und Staphylococcus aureus eine hemmende Wirkung. In der CN 106 175 996 A wird ebenfalls eine TiN/Ag-Beschichtung offenbart, die schichtweise abwechselnd aus Ti und Ag aufgebaut ist. Der Silberanteil beträgt 5×10¹⁴ bis 5×10¹⁸ Ionen/cm².

### ZUSAMMENFASSUNG DER ERFINDUNG

Folglich war es Aufgabe der vorliegenden Erfindung, eine Beschichtung für eine Wirbelsäulenimplantatkomponentenoberfläche bereitzustellen, die einer Infektion in situ vorbeugt. Insbesondere war es Aufgabe der Erfindung, eine Beschichtung für eine Wirbelsäulenimplantatkomponenten-Oberfläche bereitzustellen, die der Ansiedlung von Erregern, insbesondere von Staphylococcus epidermidis, entgegenwirkt. Auch war es Ziel, dass die beschichtete Wirbelsäulenimplantatkomponenten-Oberfläche keine Allergien oder Überempfindlichkeiten beim Patienten hervorruft. Weiterhin sollte die beschichtete Wirbelsäulenimplantatkomponenten-Oberfläche etwaigen mechanischen Einflüssen, die insbesondere während des operativen Eingriffs bei der Implantation auftreten, widerstehen können.

Zur Lösung dieser Aufgaben wird eine eine Beschichtung aufweisende Wirbelsäulenimplantatkomponente nach Anspruch 1 und ein Verfahren zum Aufbringen dieser Beschichtung auf eine Wirbelsäulenimplantatkomponente nach Anspruch 9 bereitgestellt.

So stellt die Erfindung eine Komponente eines Wirbelsäulenimplantats mit einer Beschichtung bereit, wobei die Beschichtung eine keramische Titannitridbeschichtung ist, die neben einem At%-Anteil Ti und einem At%-Anteil N einen At%-Anteil von 5-30 At% Ag aufweist.

Ein derartiger Anteil an Silber kann gegen eine durch Erreger hervorgerufene Infektion vorbeugen. Es hat sich insbesondere eine antimikrobielle Wirkung gegen Staphylococcus epidermidis gezeigt.

Zudem kann der At%-Anteil Ag, d. h. der Silberanteil, gleichzeitig so gering gehalten werden, dass die mechanische Widerstandsfähigkeit der Beschichtung für die bei der Implantation auftretenden mechanischen Kontaktkräfte ausreicht. Mit anderen Worten wird die Beschichtung des Implantats während der Implantation nicht beschädigt. Dies trifft insbesondere beim Einsetzen des beschichteten Implantats in Knochengewebe oder Verschrauben des Implantats mit Knochengewebe zu. Es wird vermutet, dass die mechanische Widerstandsfähigkeit auf einem derartigen Niveau bleibt, da der Silberanteil zwar die Härte der Beschichtung herabsetzt, dafür aber die Duktilität der Beschichtung erhöht. Damit kann verhindert werden, dass das unter der Beschichtung befindliche Implantatmaterial beziehungsweise Basismaterial des Implantats mit Körpergewebe eines Patienten in Kontakt kommt und dabei möglicherweise zu einer Überempfindlichkeit führt.

Durch ihre Widerstandsfähigkeit ist die keramische Titannitridbeschichtung mit Silberanteil, d. h. Titannitrid-Silber-Beschichtung, bei Wirbelsäulenimplantaten von Vorteil. Bei einer bevorzugten Ausführungsform weist die Beschichtung 5-20 At% Ag, 8-15 At% Ag, 8-10 At% Ag oder in etwa 10 At% Ag auf.

Diese bevorzugten Silberanteile in der Beschichtung erfüllen die obigen Anforderungen. In Bezug auf die Härte gilt, dass, je niedriger der Silberanteil ist, desto höher die Härte ist. Allerdings wirkt sich eine Verminderung der Härte, wie oben beschrieben, nicht wesentlich auf die Widerstandsfähigkeit der Beschichtung für eine Implantation des beschichteten Implantats aus.

Bei einer besonders bevorzugten Ausführungsform sind an der Beschichtungsoberfläche Silber und Titannitrid nebeneinanderliegend ausgebildet oder angeordnet.

Hierdurch kann der Ag-Anteil seine infektionshemmende Wirkung entfalten. Um diesem an der Oberfläche nebeneinander liegenden Aufbau der Beschichtung und damit direkten Kontakt mit dem Gewebe oder Flüssigkeiten des Patienten zu erreichen, werden die beiden Beschichtungskomponenten zumindest partiell gleichzeitig aufgebracht. Durch dieses gleichzeitige Aufbringen liegen die Beschichtungskomponenten zudem im Wesentlichen gleichmäßig verteilt auf der Implantatoberfläche vor.

Bei einer weiteren bevorzugten Ausführungsform der Beschichtung weist diese eine Dicke von 2,5-6 µm, 3,5-5,5 µm oder in etwa 4,5 µm auf.

Es wurde festgestellt, dass bei diesen Beschichtungsstärken zumindest einer partiellen Zerstörung der Beschichtung vermieden wird. Dabei ist eine dickere Beschichtung tendenziell von Vorteil, insbesondere wenn sich der Silberanteil in der Beschichtungsschicht in dem oberen Abschnitt der oben angegebenen Bereiche bewegt. Eine über diese Werte hinausgehende Dicke bringt hingegen keine wesentlichen Verbesserungen, sondern kann sogar eine Inhomogenität und Delamination der Beschichtung begünstigen. Es wird vermutet, dass die mechanische Widerstandsfähigkeit der Beschichtung bei den angegebenen Dicken auch dadurch bedingt ist, dass keine in der Dickenrichtung durchgehenden Abschnitte aus Silber entstehen. Mit anderen Worten liegt durch die dreidimensionale heterogene Struktur der Titannitrid-Silber-Beschichtung eine im Prinzip durchgehende Titannitridbeschichtung vor.

Bei einer weiteren bevorzugten Ausführungsform liegt die Titannitrid-Beschichtung im Wesentlichen als stöchiometrische TiN-Schicht vor.

Eine solche Schicht bildet zusammen mit dem Silberanteil eine besonders gleichmäßige inerte Schicht mit antimikrobieller Wirkung aus und wirkt damit sowohl einer Überempfindlichkeit als auch einer Infektion nach Implantation des Implantats vor.

Zudem stellt die vorliegende Erfindung zumindest eine Implantatkomponente von einem Wirbelsäulenimplantat bereit, die zumindest abschnittsweise mit einer Beschichtung nach einem der vorstehenden Ansprüche beschichtet ist.

Wie oben beschrieben, kann es insbesondere durch die vor der Implantation der Umgebung ausgesetzten Implantate zu Infektionen kommen. Dabei wurde festgestellt, dass besonders im Wirbelsäulenbereich solche Infektionen auf den Erreger Staphylococcus epidermidis zurückzuführen sind. Deswegen ist die vorbeugende Wirkung der Beschichtung insbesondere hier wirkungsvoll.

Es ist hervorzuheben, dass auch eine Beschädigung der Beschichtung, wenn überhaupt, nur sehr geringe Auswirkungen auf die infektionshemmende Wirkung der Beschichtung hat.

Bei einer bevorzugten Ausführungsform ist die Implantatkomponente zumindest eine Komponente eines Spondylodeseimplantats oder ein Wirbelsäulencage.

Bei einem Spondylodeseimplantat werden bevorzugt sämtliche Teile, wie z. B. Verbindungselemente, Stangen und Schrauben mit der vorliegenden Beschichtung versehen. Auch Cages sind sehr gut für den Einsatz dieser Beschichtung geeignet, da sie regelmäßig eine komplexe und verwinkelte Geometrie aufweisen. Zudem können Wirbelsäulencages Verankerungselemente aufweisen, die in das Knochengewebe der Wirbelkörper eingreifen und entsprechend belastet werden, wenn ein solcher Wirbelsäulencage zwischen zwei Wirbelkörpern eingesetzt wird.

Bei einer weiteren besonders bevorzugten Ausführungsform weist die Oberfläche des beschichteten Abschnitts der Implantatkomponente Titannitrid mit darin eingebetteten Ag-Inseln oder Silberinseln auf.

Wie oben beschrieben, kann durch eine solche Verteilung von Titannitrid und Silber in der Beschichtung eine im Wesentlichen durchgehende Titannitridbeschichtung bereitgestellt werden. Als Ergebnis wird eine ausreichende mechanische Widerstandsfähigkeit einer solchen Beschichtung erreicht.

Bei einer weiteren Ausführungsform weist die zu beschichtende Implantatkomponente eine Titanlegierung auf und besteht bevorzugt aus dieser.

Zum einen haftet die Titannitrid-Silber-Beschichtung aufgrund des Titannitridanteils besonders gut auf einer Titanlegierung so einer Implantatkomponente und beugt so einer durch Ablösen verursachten Beschädigung der Beschichtung vor. Zum anderen reduziert eine zu beschichtende Implantatkomponente aus einer Titanlegierung das Risiko einer Überempfindlichkeit noch weiter, da eine solche Überempfindlichkeit selbst bei einer Beschädigung der Beschichtung aufgrund der Biokompatibilität von Titan unwahrscheinlich ist.

Weiterhin stellt die vorliegende Erfindung ein Verfahren zum Aufbringen einer Beschichtung auf eine Wirbelsäulenimplantatkomponente bereit, das als Schritte ein Bereitstellen einer zu beschichtenden Implantatkomponente für ein Wirbelsäulenimplantat in einer Beschichtungskammer, Bereitstellen von mindestens einem Target, das einen für die Beschichtung vorbestimmten At%-Anteil an Silber und Titan aufweist und/oder Bereitstellen mindestens eines Ti-Targets und mindestens eines Ag-Targets und ein Verdampfen des mindestens einen Targets in einer zumindest stickstoffhaltigen Atmosphäre und gleichzeitiges Beschichten der Implantatkomponente mit dem verdampften Metall des mindestens einen Targets umfasst.

Dieses Verfahren ermöglicht ein gleichzeitiges Beschichten mit Titannitrid und Silber zum Ausbilden der Titannitrid-Silber-Beschichtung. Dabei wird durch das gleichzeitige Beschichten sichergestellt, dass an der Oberfläche der Beschichtung Silber exponiert ist und sich damit der infektionshemmende Effekt der Beschichtung entfalten kann. Zudem kann durch eine Wahl der Anzahl der jeweiligen Targets der Beschichtungskomponenten der At%-Anteil zumindest in seiner Größenordnung auf die gewünschte Zusammensetzung der Beschichtung eingestellt werden.

Alternativ oder zusätzlich zu der Einstellung der Zusammensetzung der Beschichtung über die Anzahl der jeweiligen Targets kann mindestens ein Target, das ein vorbestimmtes Verhältnis von Titan und Silber aufweist, bereitgestellt werden. Vorzugsweise wird allerdings ausschließlich ein oder mehrere derartige Targets mit entsprechend der gewünschten Zusammensetzung der Beschichtung vorbestimmten At%-Anteilen an Titan und Silber verwendet.

Bei einer bevorzugten Ausführungsform des Verfahrens wird das Verdampfen des mindestens einen Targets durch Lichtbogenverdampfen ausgeführt, wobei die an den Targets angelegte Spannung 15-30 V oder 20-25 V und der angelegte Strom 40-70 A betragen.

Diese Einstellbereiche der Spannung und des Stroms ermöglichen wie die oben genannte Anzahl der Targets der jeweiligen Beschichtungskomponente eine Einstellung der Beschichtungszusammensetzung. Dabei kann vor allem eine feinere Einstellung als bei der Wahl der Anzahl der Targets erreicht werden.

Bei einer weiteren Ausführungsform des Verfahrens wird nach dem Bereitstellen des zu beschichtenden Implantats in der Beschichtungskammer eine Reinigung der zu beschichtenden Implantatoberfläche durch eine Glimmentladung unter einer Wasserstoffatmosphäre ausgeführt.

Dieser Reinigungsschritt hat den Vorteil, dass etwaige an der Oberfläche des zu beschichtenden Implantats vorliegende organischen Rückstände entfernt werden und so die Haftung der Beschichtung auf dem Implantat verbessert wird.

Bei einer weiteren bevorzugten Ausführungsform des Verfahrens wird nach dem Einbringen des zu beschichtenden Implantats in die Beschichtungskammer eine Reinigung der zu beschichtenden Implantatoberfläche durch Beschießen der Implantatoberfläche mit Ionen unter einer inerten Atmosphäre durchgeführt.

Hierdurch wird eine möglicherweise vorhandene Oxidschicht an der Implantatoberfläche entfernt, die anderweitig die Haftung der Beschichtung an dem Implantat reduzieren würde.

Eine derartige Oxidschicht bildet sich zum Beispiel bei Implantaten aus Titanlegierungen und kann insbesondere durch Beschießen mit Titanionen durch den Verfahrensschritt dieser Ausführungsform entfernt werden. Die Titanionen können dabei vorzugsweise über ein für die Beschichtung zu verwendendes Ti-Target erzeugt werden. Dabei wirkt die inerte Atmosphäre, wie zum Beispiel eine Argonatmosphäre, einer erneuten Ausbildung einer Oxidschicht entgegen.

Bereitgestellt (nicht erfindungsgemäß) wird eine Verwendung der Beschichtung zum Vorbeugen gegen einen Biofilm auf einer Wirbelsäulenimplantatkomponente.

Eine derartige Verwendung der Beschichtung ist, wie oben bereits beschrieben, besonders vorteilhaft.

### AUSFÜHRLICHE ERLÄUTERUNG BEVORZUGTER AUSFÜHRUNGSFORMEN

Unter einer Beschichtung ist im Rahmen der vorliegenden Erfindung eine durch ein technisches Verfahren aufgebrachte Beschichtung zu verstehen. Beispiele solcher technischer Verfahren sind die chemische Gasphasenabscheidung (CVD - Chemical Vapor Deposition), physikalische Gasphasenabscheidung (PVD - Physical Vapor Deposition) oder galvanische Beschichtung.

Wie oben beschrieben umfasst eine Beschichtung eine Mischung aus Titannitrid und Silber, die hier auch als Titannitrid-Silber-Beschichtung bezeichnet wird. Mit anderen Worten weist die Beschichtung mindestens eine einzelne Schicht einer keramischen Titannitridbeschichtung auf, in der Silber eingebettet ist. Dabei liegt das Silber in Form von Silberinseln vor, d. h. Silber beziehungsweise Silberatome sind neben dem Titannitridgitter angeordnet. Aufgrund der Größe der Silberatome wird davon ausgegangen, dass, wenn überhaupt, nur ein geringer Anteil des Silbers interstitiell in dem Titannitridgitter angeordnet ist. Stattdessen wurde beobachtet, dass das Silber in Form von Silberagglomeraten in der Titannitrid-Silber-Beschichtung vorliegt. Mit anderen Worten liegt das Silber in dem Titannitridgitter vor, ist aber nicht in dieses integriert. Diese Silberagglomerate liegen vorzugsweise in einem Bereich von 1 µm bis 50 µm und noch bevorzugter in einem Bereich von 5 µm bis 30 µm vor.

Des Weiteren wird davon ausgegangen, dass die Wirksamkeit des Silbers insbesondere dadurch entsteht, dass das Silber im implantierten Zustand des Implantats bei Kontakt mit Körperflüssigkeit Lokalelementbildung in den ionischen Zustand übergeht und so seine antimikrobielle Wirkung entfaltet. Dass diese Lokalelementbildung stattfinden kann, wird durch eine Anordnung dieser Inseln an der Oberfläche der Beschichtung ermöglicht. Erreicht wird diese Anordnung durch ein zumindest partiell gleichzeitiges Beschichten des Implantats mit Titannitrid und Silber.

Die Beschichtung weist durch ihre antimikrobiellen Eigenschaften eine infektionshemmende Wirkung auf, die sich insbesondere auch auf Staphylococcus epidermidis bezieht. Es wird vermutet, dass der in der Titannitridmatrix vorliegende Silberanteil der Beschichtung die Ausbildung eines Biofilms stört, den diese Bakterien entwickeln. Durch diese Störung funktioniert der durch diesen Biofilm hervorgerufene Schutzmechanismus der Bakterien vor Antibiotika zumindest nicht mehr ausreichend.

Weiterhin wurde beobachtet, dass sich das Silber in Ionenform aus der Beschichtung lösen kann. Es wird davon ausgegangen, dass diese an der Oberfläche der Beschichtung ionisierten Silberpartikel in unmittelbarer Umgebung des Implantats eine Wirkzone ("inhibition zone") ausbilden, in der sie eine antimikrobielle Wirkung entfalten. Folglich kann mit der Beschichtung nicht nur gegen eine sich unmittelbar von der Oberfläche des Implantats ausbreitenden Infektion vorgebeugt werden.

Eine Titannitrid-Silber-Beschichtung mit einem Silberanteil von 5-30 At-% entfaltet eine antimikrobielle Wirkung. Dabei liegt insbesondere eine Wirksamkeit gegen Staphylococcus epidermidis vor. Wie oben beschrieben, ist dieser Erreger üblicherweise auf der Haut von Menschen zu finden und vermutlich deswegen in vielen Fällen Ursache für eine nach der Implantation entstehende Infektion. Dabei deuten Untersuchungen darauf hin, dass insbesondere im Wirbelsäulenbereich ein erhöhtes Risiko besteht, dass dieser Erreger nach Implantation eine Infektion auslöst. Dies wird möglicherweise dadurch begünstigt, dass der Staphylococcus epidermidis eine vergleichsweise geringe Virulenz unter den Staphylokokken aufweist. Das führt dazu, dass die Infektionsanzeichen später auftreten und anfangs somit möglicherweise übersehen werden. Da die Beschichtung gerade gegen diesen Erreger eine Wirkung zeigt, kann insbesondere einer aus dem vorgenannten Grund häufig sehr spät erkannten Infektion vorgebeugt werden.

Vorzugsweise ist der At%-Anteil an Silber geringer als der At%-Anteil an Titan. Mit anderen Worten ist es nicht erforderlich, dass eine stöchiometrische Verteilung vorliegt. Die Verteilung kann über- oder unterstöchiometrisch sein. In Summe weist die Beschichtung einen Anteil von mindestens 70 At-% Titannitrid auf.

Durch einen maximalen Silberanteil von 30 At% wird sichergestellt, dass das Titannitrid Einlagerungen von Silber oder Silberinseln aufweist und nicht umgekehrt. Dies hat den Vorteil, dass das Titannitrid eine im Wesentlichen durchgehende Beschichtung bereitstellt in der das Silber eingelagert ist. Ein Herauslösen von Silber hat somit im Allgemeinen keinen negativen Einfluss auf die Funktionalität der Beschichtung. Wie bereits beschrieben, erweitert sich hierdurch sogar tendenziell der antimikrobielle Schutzbereich.

Auch andere bevorzugte Silberanteile für die vorliegende Beschichtung, wie zum Beispiel ein Silberanteil von 5-20 At%, 8-15 At%, 8-10 At% oder in etwa 10 At% weisen diesen Vorteil auf. Unter anderem führt dieser Aufbau der Beschichtung dazu, dass zumindest ein Teil des Silberanteils an der Beschichtungsoberfläche neben dem Titannitridanteil vorliegt.

Der Silberanteil führt zusammen mit dem keramischen Titannitridanteil als Titannitrid-Silber-Beschichtung neben der oben erwähnten antimikrobiellen Wirkung zudem zu einer Veränderung der mechanischen Eigenschaften im Verhältnis zu einer reinen Titannitridbeschichtung. Insbesondere wird die Beschichtung durch diesen Aufbau weicher.

Es wird vermutet, dass aufgrund einer damit einhergehenden Duktilität ihre mechanische Widerstandsfähigkeit bzw. Festigkeit weiter ausreicht, um den bei einer Implantation des Implantats auftretenden mechanischen Einflüssen zu widerstehen. Solche mechanischen Einflüsse entstehen zum Beispiel beim Erzeugen eines Presssitzes eines Implantats im Knochengewebe, durch Kontakt einer Implantatkomponente mit einem Befestigungselement, wie zum Beispiel beim Eindrehen von Knochenschrauben zur Befestigung einer Platte, oder bei der Montage mit einer anderen Implantatkomponente, wie zum Beispiel bei einem Spondylodeseaufbau.

Aus diesem Grund ist die vorliegende Beschichtung insbesondere für Implantate geeignet, welche nach Implantation das Skelett eines Patienten unterstützen oder Teile dieses Skeletts ersetzen. Bei solchen Implantaten tritt eine mechanische Belastung der Beschichtung im Allgemeinen während der Implantation und des Zusammenbaus eines Implantats auf. Nach Implantation entstehen insbesondere durch die alltägliche Belastung des Implantats im Körper eines Patienten Spannungen und Dehnungen in der Beschichtung. Auch hier ist die vorliegende Beschichtung von Vorteil.

Mit anderen Worten ist die Beschichtung vor allem für Implantate geeignet, bei denen Abrieb vor allem während der Implantation und/oder Zusammenbau des Implantats besteht. Anders ausgedrückt erfährt die Beschichtung im implantierten Zustand im Wesentlichen keinen funktionsbedingten Abrieb. Es wurde festgestellt, dass hierfür eine Dicke der Beschichtung unter 10 insbesondere von 2,5-6 bevorzugt 3,5-5,5 µm und noch bevorzugter in etwa 4,5 ausreichend ist. Allerdings ist es denkbar eine derartige Beschichtung auch mit einer höheren Schichtdicke zu verwenden.

Weiterhin kann bei der vorliegenden Beschichtung durch den Silberanteil teilweise der Unterschied der Materialeigenschaften, insbesondere die Elastizität, zu dem darunterliegenden Basismaterial des Implantats geringer sein. Auch dadurch wird eine ausreichende mechanische Widerstandsfähigkeit und Haftung der Beschichtung ermöglicht. Auch kann die Beschichtung unter anderem deswegen auf eine große Vielfalt unterschiedlicher Implantatmaterialien aufgebracht werden.

Zusammenfassend zeigt die Titannitrid-Silber-Schicht der Beschichtung somit sowohl vorteilhafte antimikrobielle als auch mechanische Eigenschaften, die für eine mit dieser Beschichtung zumindest abschnittsweise beschichtete Wirbelsäulenimplantatkomponente von Nutzen sind.

Erfindungsgemäß wird eine derartige Beschichtung durch die oben bereits genannte physikalische Gasabscheidung (PVD-Verfahren) hergestellt. Vor dem Einbringen in die Beschichtungskammer wird das mit der Beschichtung zu versehende Implantat wässrig gereinigt.

Dann wird das Implantat in die Beschichtungskammer eingeführt, die anschließend evakuiert wird. Für die nachfolgenden Prozesse wird das Implantat vorzugsweise auf 400 bis 600 °C aufgeheizt, um die Beweglichkeit von Ionen an der Oberfläche des Implantats zu verbessern und eine bessere Haftung der Beschichtung auf dem Implantat zu erreichen.

Vorzugsweise erfolgt eine weitere Reinigung der Implantatoberfläche innerhalb der Beschichtungskammer und vor dem Aufbringen der Beschichtung. Zum Beispiel kann eine Reinigung mittels einer Glimmentladung in einer Wasserstoffatmosphäre durchgeführt werden, um etwaige organische Rückstände auf der unbeschichteten Implantatoberfläche zu entfernen.

Weiterhin ist es möglich, eine Reinigung der Implantatoberfläche mittels Ionenätzens durchzuführen. Hierbei wird das Implantat unter einer inerten Atmosphäre, insbesondere einer Argonatmosphäre, mit Ionen (z. B. Titanionen, Argonionen) beschossen, um eine an der Oberfläche des unbeschichteten Implantatmaterials möglicherweise vorhandene Oxidschicht zu entfernen. Auch hierdurch wird eine bessere Haftung der Beschichtung an der Oberfläche des Implantats erreicht.

Die genannten Reinigungsschritte finden vorzugsweise in einer Unterdruckatmosphäre von 10⁻¹ bis 10⁻⁴ mbar statt.

Nach dieser optionalen Reinigung erfolgt das Aufbringen der Beschichtung auf das Implantat unter einer Stickstoffatmosphäre.

Wie oben bereits beschrieben kann diese Beschichtung entsprechend ihrer gewünschten Zusammensetzung mit mindestens einem Silbertarget und mindestens einem Titantarget ausgeführt werden. Genauso ist es möglich, ein oder mehrere Targets zu verwenden, das die für die Beschichtung vorgesehenen At%-Anteile an Silber und Titan aufweist. Hierbei wird folglich die Zusammensetzung der Beschichtung durch die Zusammensetzung des mindestens einen Targets bestimmt.

Um eine Streuung des verdampften Targetmaterials an Gasteilchen in der Beschichtungskammer und damit den Verlust an Targetmaterial möglichst gering zu halten, erfolgt die Beschichtung unter einem Unterdruck in einem Bereich von 10⁻² bis 10⁻³ mbar.

Ist die gewünschte Atmosphäre eingestellt, beginnt der Verdampfungsprozess des mindestens einen Targets. Besonders bevorzugt wird hierfür ein Lichtbogen verwendet, der durch einen starken Strom mittels elektrischer Entladung Material aus den Targets herauslöst und in die Gasphase überführt. Bei dieser Entladung werden insbesondere Spannungen in einem Bereich von 15-30 V und bevorzugt in einem Bereich von 20-25 V sowie Ströme in einem Bereich von 40-70 A eingesetzt. Dem Fachmann ist es allerdings verständlich, dass auch andere Verfahren zum Verdampfen der Targets verwendet werden können, wie zum Beispiel thermisches Verdampfen, Elektronenstrahlverdampfen oder Laserstrahlverdampfen.

Zumindest während eines Teils des Beschichtungsvorgangs erfolgt bei Verwendung von Targets mit unterschiedlichen Materialien die Beschichtung mit dem Silbertarget und dem Titantarget gleichzeitig, um die oben beschriebene Struktur der Titannitrid-Silber-Beschichtung zu erzeugen.

Je nach zu beschichtendem Basismaterial des Implantats kann an diesem zudem eine negative Spannung von 100 V bis 1500 V angelegt werden, um die Haftung und Schichthomogenität zu verbessern. Auch können, um eine möglichst gleichmäßige Beschichtung zu erreichen, die Targets und das Implantat während des Beschichtungsvorgangs relativ zueinander bewegt werden.

Nach erfolgter Beschichtung und einer Abkühlphase wird die Beschichtungskammer wieder belüftet und die beschichtete Wirbelsäulenimplantatkomponente bzw. die beschichteten Wirbelsäulenimplantatkomponenten können entnommen werden. Die Abkühlung erfolgt vorzugsweise mit Unterstützung einer Gasatmosphäre (z. B. Stickstoff oder ein inertes Gas) für eine verbesserte Wärmeabfuhr, sodass der Abkühlungsvorgang beschleunigt wird.

## Patentansprüche

1. Wirbelsäulenimplantatkomponente, die zumindest abschnittsweise eine Beschichtung aufweist, wobei die Beschichtung eine keramische Titannitridbeschichtung ist, die neben einem At%-Anteil Ti und einem At%-Anteil N einen At%-Anteil von 5-30 At% Ag aufweist.

2. Wirbelsäulenimplantatkomponente nach Anspruch 1, wobei die Beschichtung 5-20 At% Ag, 8-15 At% Ag, 8-10 At% Ag oder in etwa 10 At% Ag aufweist.

3. Wirbelsäulenimplantatkomponente nach Anspruch 1 oder 2, wobei auf der Beschichtungsoberfläche Ag und Titannitrid nebeneinanderliegend ausgebildet sind.

4. Wirbelsäulenimplantatkomponente nach einem der vorstehenden Ansprüche, wobei die Beschichtung eine Dicke von 2,5-6 3,5-5,5 µm oder in etwa 4,5 µm aufweist.

5. Wirbelsäulenimplantatkomponente nach einem der vorstehenden Ansprüche, bei der die Titannitrid-Beschichtung im Wesentlichen als stöchiometrische TiN-Schicht vorliegt.

6. Wirbelsäulenimplantatkomponente nach einem der vorstehenden Ansprüche, wobei die Wirbelsäulenimplantatkomponente zumindest eine Komponente eines Spondylodeseimplantats oder ein Cage ist.

7. Wirbelsäulenimplantatkomponente nach einem der vorstehenden Ansprüche, bei welcher die Oberfläche des beschichteten Abschnitts Titannitrid mit darin eingebetteten Ag-Inseln aufweist.

8. Wirbelsäulenimplantatkomponente nach einem der vorstehenden Ansprüche, wobei die zu beschichtende Implantatkomponente eine Titanlegierung aufweist und bevorzugt aus dieser besteht.

9. Verfahren zum Aufbringen einer Implantatbeschichtung nach einem der vorstehenden Ansprüche auf eine Wirbelsäulenimplantatkomponente nach einem der vorstehenden Ansprüche , das die Schritte umfasst:
- Bereitstellen einer zu beschichtenden Implantatkomponente, insbesondere einer Implantatkomponente für ein Wirbelsäulenimplantat in einer Beschichtungskammer;
- Bereitstellen von mindestens einem Target, das einen für die Beschichtung vorbestimmten At%-Anteil an Silber und Titan aufweist und/oder Bereitstellen mindestens eines Ti-Targets und mindestens eines Ag-Targets;
- Bereitstellen einer zumindest stickstoffhaltigen Atmosphäre;
- Verdampfen des mindestens einen Targets; und
- gleichzeitiges Beschichten der Implantatkomponente mit dem verdampften Metall des mindestens einen Targets.

10. Verfahren zum Aufbringen einer Implantatbeschichtung nach Anspruch 9, wobei das Verdampfen des mindestens einen Targets durch Lichtbogenverdampfen ausgeführt wird und dabei die an den Targets angelegte Spannung 15-30 V oder 20-25 V und der angelegte Strom 40-70 A betragen.

11. Verfahren nach Anspruch 9 oder 10, bei dem nach dem Bereitstellen des zu beschichtenden Implantats in der Beschichtungskammer eine Reinigung der zu beschichtenden Implantatoberfläche durch eine Glimmentladung unter einer Wasserstoffatmosphäre ausgeführt wird.

12. Verfahren nach einem der Ansprüche 9 bis 11, bei dem nach dem Einbringen des zu beschichtenden Implantats in die Beschichtungskammer eine Reinigung der zu beschichtenden Implantatoberfläche durch Beschießen der Implantatoberfläche mit Ionen unter einer inerten Atmosphäre durchgeführt wird.

## Claims

1. A spinal implant component having a coating at least in sections, wherein the coating is a ceramic titanium nitride coating, which in addition to an At% content Ti and an At% content N, has an At% content of 5-30 At% Ag.

2. The spinal implant component according to claim 1, wherein the coating comprises 5-20 At% Ag, 8-15 At% Ag, 8-10 At% Ag, or approximately 10 At% Ag.

3. The spinal implant component according to claim 1 or 2, wherein Ag and titanium nitride are formed side by side on the coating surface.

4. The spinal implant component according to any of the foregoing claims, wherein the coating has a thickness of 2.5-6 3.5-5.5 µm or approximately 4.5 µm.

5. The spinal implant component according to any of the foregoing claims, in which the titanium nitride coating is present substantially as a stoichiometric TiN layer.

6. The spinal implant component according to any of the foregoing claims, wherein the spinal implant component is at least a component of a spinal fusion implant or a cage.

7. The spinal implant component according to any of the foregoing claims, in which the surface of the coated section comprises titanium nitride with Ag islands embedded therein.

8. The spinal implant component according to any of the foregoing claims, wherein the implant component to be coated comprises a titanium alloy and preferably consists of this.

9. A method for applying an implant coating according to any of the foregoing claims on a spinal implant component according to any of the foregoing claims, comprising the steps:
- Provision of an implant component to be coated, in particular, an implant component for a spinal implant in a coating chamber;
- Provision of at least one target, which comprises a predetermined At% content of silver and titanium for the coating and/or provision of at least one Ti target and at least one Ag target;
- Provision of an at least nitrogen containing atmosphere;
- Vaporization of the at least one target; and
- Simultaneous coating of the implant component having the vaporized metal of the at least one target.

10. The method for applying an implant coating according to claim 9, wherein the vaporization of the at least one target is carried out by arc evaporation and, in the process, the voltage applied to the targets is 15-30 V or 20-25 V and the applied current is 40-70 A.

11. The method according to claim 9 or 10, in which after the provision of the implant to be coated in the coating chamber a cleansing of the implant surface to be coated is carried out by means of a glow discharge under a hydrogen atmosphere.

12. The method according to any of claims 9 to 11, in which after bringing the implant to be coated into the coating chamber a cleansing of the implant surface to be coated is carried out by means of bombarding the implant surface with ions under an inert atmosphere.

## Revendications

1. Composant d'implant de colonne vertébrale, qui présente au moins par tronçon un revêtement, dans lequel le revêtement est un revêtement de nitrure de titane céramique qui présente, en plus d'une proportion en % atomique de Ti et d'une proportion en % atomique de N, une proportion en % atomique de 5 à 30 % atomique d'Ag.

2. Composant d'implant de colonne vertébrale selon la revendication 1, dans lequel le revêtement présente 5 à 20 % atomique d'Ag, 8 à 15 % atomique d'Ag, 8 à 10 % atomique d'Ag ou environ 10 % atomique d'Ag.

3. Composant d'implant de colonne vertébrale selon la revendication 1 ou 2, dans lequel de l'Ag et du nitrure de titane sont formés côte à côte sur la surface du revêtement.

4. Composant d'implant de colonne vertébrale selon l'une des revendications précédentes, dans lequel le revêtement présente une épaisseur de 2,5 à 6 3,5 à 5,5 µm ou environ 4,5 µm.

5. Composant d'implant de colonne vertébrale selon l'une des revendications précédentes, dans lequel le revêtement de nitrure de titane se présente essentiellement sous forme de couche de TiN stœchiométrique.

6. Composant d'implant de colonne vertébrale selon l'une des revendications précédentes, où le composant d'implant de colonne vertébrale est au moins un composant d'un implant de spondylodèse ou une cage.

7. Composant d'implant de colonne vertébrale selon l'une des revendications précédentes, dans lequel la surface du tronçon revêtu présente du nitrure de titane avec des îlots d'Ag incorporés à l'intérieur.

8. Composant d'implant de colonne vertébrale selon l'une des revendications précédentes, dans lequel le composant d'implant à revêtir présente un alliage de titane et se compose de préférence de celui-ci.

9. Procédé de dépôt d'un revêtement d'implant selon l'une des revendications précédentes sur un composant d'implant de colonnes vertébrales selon l'une des revendications précédentes, qui comprend les étapes de :
- fourniture d'un composant d'implant à revêtir, en particulier d'un composant d'implant pour un implant de colonne vertébrale dans une chambre de revêtement ;
- fourniture d'au moins une cible, qui présente une proportion en % atomique d'argent et de titane prédéfinie pour le revêtement et/ou fourniture d'au moins une cible en Ti et d'au moins une cible en Ag ;
- fourniture d'une atmosphère au moins contenant de l'azote ;
- vaporisation d'au moins une cible ; et
- revêtement simultané du composant d'implant avec le métal vaporisé de la au moins une cible.

10. Procédé de dépôt d'un revêtement d'implant selon la revendication 9, dans lequel la vaporisation de la au moins une cible est exécutée par vaporisation à arc lumineux et la tension appliquée aux cibles est alors de 15 à 30 V ou de 20 à 25 V et le courant appliqué de 40 à 70 A.

11. Procédé selon la revendication 9 ou 10, dans lequel, après la fourniture de l'implant à revêtir dans la chambre de revêtement, un nettoyage de la surface d'implant à revêtir est réalisé par une décharge luminescente sous une atmosphère d'hydrogène.

12. Procédé selon l'une des revendications 9 à 11, dans lequel, après introduction de l'implant à revêtir dans la chambre de revêtement, un nettoyage de la surface de l'implant à revêtir est réalisé par bombardement de la surface d'implant avec des ions sous une atmosphère inerte.
